(19) Europäisches Patentamt
European Patent Office
Office européen des brevets

(11) **EP 3 598 936 B1**

(12) **EUROPEAN PATENT SPECIFICATION**

(45) Date of publication and mention
of the grant of the patent:
**06.11.2024 Bulletin 2024/45**

(21) Application number: **19187470.0**

(22) Date of filing: **22.07.2019**

(51) International Patent Classification (IPC):
***A61B 5/00*** *(2006.01)*

(52) Cooperative Patent Classification (CPC):
**A61B 5/742; A61B 5/7221; A61B 5/7275;
G16H 50/30**

(54) **PHYSIOLOGICAL INFORMATION MEASUREMENT DEVICE AND METHOD FOR OUTPUTTING DATA FOR DISPLAYING PHYSIOLOGICAL INFORMATION**

MESSGERÄT FÜR PHYSIOLOGISCHE INFORMATION UND VERFAHREN ZUR AUSGABE UND DARSTELLUNG PHYSIOLOGISCHER INFORMATION

DISPOSITIF DE MESURE D'INFORMATIONS PHYSIOLOGIQUES ET PROCÉDÉ DE PRODUCTION ET DE PRÉSENTATION D'INFORMATIONS PHYSIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2018 JP 2018139512**

(43) Date of publication of application:
**29.01.2020 Bulletin 2020/05**

(73) Proprietor: **Nihon Kohden Corporation
Tokyo 161-8560 (JP)**

(72) Inventors:
• **UEDA, Yoshinori
Tokorozawa-shi, Saitama (JP)**
• **ITO, Kazumasa
Tokorozawa-shi, Saitama (JP)**
• **FUJISAKI, Hideki
Tokorozawa-shi, Saitama (JP)**

(74) Representative: **Grünecker Patent- und Rechtsanwälte
PartG mbB
Leopoldstraße 4
80802 München (DE)**

(56) References cited:
**US-A1- 2009 088 606      US-A1- 2011 004 085
US-A1- 2011 196 213**

**Description**

CROSS REFERENCE TO RELATED APPLICATION

This application is based on Japanese Patent Application No. 2018-139512 filed on July 25, 2018.

BACKGROUND

The presently disclosed subject matter relates to a device for displaying physiological information of a subject and a method for outputting data for displaying the physiological information.

JP-A-2015-107229 discloses a device for displaying physiological information. In this device, various sets of physiological information of a subject are displayed as numerical values. Usually, such a displayed numerical value corresponds to a result which is obtained by performing a time-averaging process on time-varying values of physiological information for a predetermined time period.

The amount of a temporal change of the values of physiological information may be used as an index indicating the instability of physiological information (i.e., the condition of the subject). However, the amount of a temporal change of physiological information (the level of variation from the displayed value) may not be recognized from the displayed value itself which is obtained through a time-averaging process.

US 2011/0196213 A1 provides a display for biological values. Biological measurements are grouped by month so that a patient's progress can be monitored.

US 2011/0004085 A1 deals with performance reports associated with continuous sensor data from multiple analysis time periods. In an exemplary performance report, two line graphs illustrate average blood glucose levels for each hour of a 24-hour period over the course of the relevant analysis time periods.

SUMMARY OF THE INVENTION

The invention is defined by the appended claims.

SUMMARY OF THE DISCLOSURE

Accordingly, the presently disclosed subject matter is to assist a user to accurately recognize the condition of the subject.

According to a first aspect of the presently disclosed subject matter, there is provided a physiological information measurement device including:

   one or more processors configured to calculate a first time-averaged value of physiological information of a subject for a first time period and calculate a second time-averaged value of the physiological information for a second time period that is different from the first time period; and
a display configured to display a first graph curve indicating a temporal change of the first time-averaged value and a second graph curve indicating a temporal change of the second time-averaged value.

According to a second aspect of the presently disclosed subject matter, there is provided a method for outputting data for displaying physiological information, the method including:

   acquiring physiological information of a subject;
   calculating a first time-averaged value of the physiological information for a first time period;
   calculating a second time-averaged value of the physiological information for a second time period that is different from the first time period; and
   outputting data for displaying a first graph curve indicating a temporal change of the first time-averaged value and a second graph curve indicating a temporal change of the second time-averaged value.

According to the above-described configuration, the user can check, through the display, time changes of a plurality of time-averaged values which are calculated from the same physiological information that is acquired from the subject, for respective different time periods. A graph curve indicating a temporal change of a time-averaged value which is calculated for a shorter time period reflects more strongly the variation of acquired physiological information. Therefore, when the consistency between the first and second graph curves is low, it is indicated that the physiological information of the subject is unstable. That is, the user can recognize, through the display, not only the time-averaged value of the physiological information of the subject, but also the stability of the physiological information. Therefore, it is possible to assist the user to accurately recognize the condition of the subject.

BRIEF DESCRIPTION OF DRAWINGS

   FIG. 1 illustrates the functional configuration of a pulse oximeter according to an exemplary embodiment.
   FIG. 2 illustrates the procedure of a process which is performed by the pulse oximeter of FIG. 1.
   FIG. 3 illustrates a display example of a display of the pulse oximeter of FIG. 1.
   FIG. 4 illustrates another display example of the display of the pulse oximeter of FIG. 1.

DESCRIPTION OF EMBODIMENTS

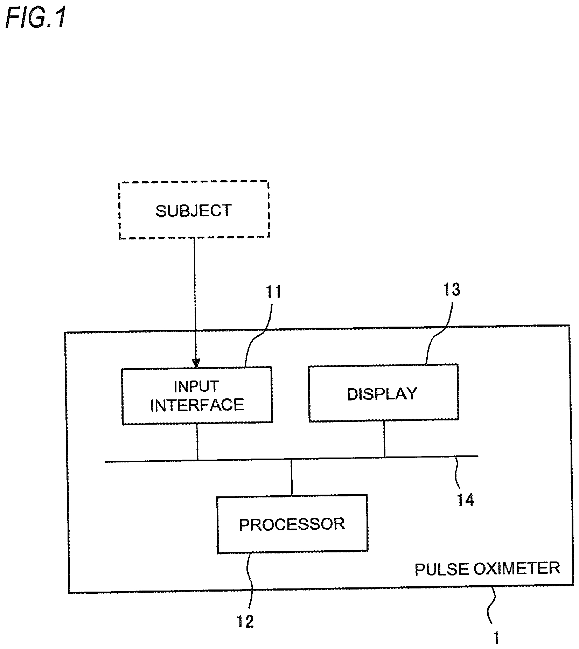

Hereinafter, an exemplary embodiment will be described in detail with reference to the accompanying drawings. FIG. 1 illustrates the functional configuration of a pulse oximeter 1 according to an exemplary embodiment.

The pulse oximeter 1 may include an input interface 11, one or more processors 12, a display 13, and a bus 14. The bus 14 interconnects the input interface 11, the processor 12, and the display 13 such that signals and data can be exchanged between these components.

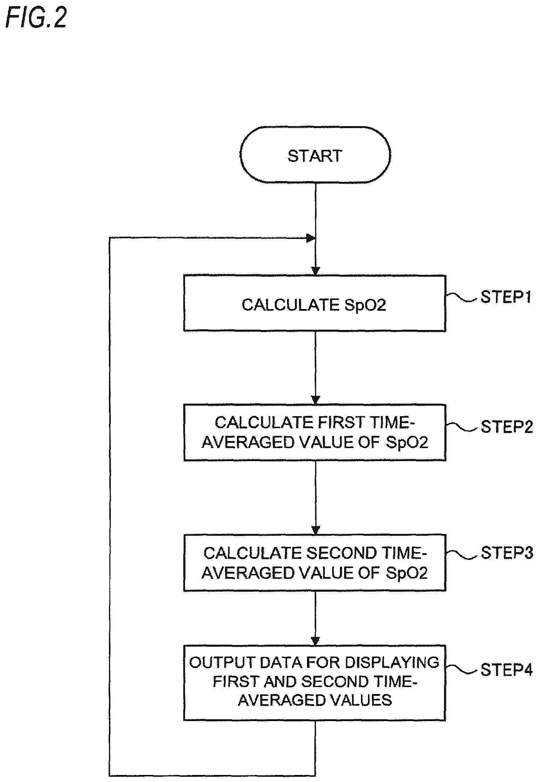

A probe (not illustrated) is attached to the fingertip or earlobe of a subject. The probe may include a light emitter and a light detector. The light emitter emits a red light beam and an infrared light beam. The light detector outputs a signal corresponding to the amounts of the red and infrared light beams which are transmitted through or reflected from a portion where the probe is attached. The signal is supplied to the input interface 11. The input interface 11 may include an appropriate circuit configuration that can convert the supplied signals to data on which the processor 12 can execute a process illustrated in FIG. 2.

The processor 12 is configured to calculate at least the transcutaneous arterial oxygen saturation (SpO2) of the subject based on the data which are supplied through the input interface 11 (STEP 1). The method for calculating the SpO2 is known, and therefore, its detailed description will be omitted. The SpO2 is an example of the physiological information, and the calculation of the SpO2 is an example of acquiring of the physiological information. The calculation of the SpO2 is performed every predetermined time period. For example, the predetermined time period is one second.

Every time when the SpO2 is calculated, the processor 12 calculates a first time-averaged value A1 of the SpO2 for a first time period T1 (STEP 2). Specifically, the average value of a plurality of the SpO2 values which are calculated during a time period of the first time period T1 before the present time is calculated. For example, the first time period T1 is 10 seconds. When the time-varying SpO2 is expressed as f(t), the first time-averaged value A1(t0) at the present time t0 can be indicated by the following expression.

$$A1(to) = \frac{1}{T1} \int_{to-T1}^{t0} f(t)dt$$

Every time when the SpO2 is calculated, the processor 12 further calculates a second time-averaged value A2 of the SpO2 for a second time period T2 (STEP 3). Specifically, the average value of a plurality of the SpO2 values which are calculated during a time period of the second time period T2 before the present time is calculated. The second time period T2 is different from the first time period T1. For example, the second time period T2 is three seconds. When the time-varying SpO2 is expressed as f(t), the second time-averaged value A2(t0) at the present time t0 can be indicated by the following expression.

$$A2(to) = \frac{1}{T2} \int_{to-T2}^{t0} f(t)dt$$

STEP 2 and STEP 3 may be conducted in the reverse order or at the same time.

Then, the processor 12 outputs data for displaying the first and second time-averaged values A1, A2 of the SpO2 on the display 13. The display 13 displays the first and second time-averaged values A1, A2 based on the data supplied from the processor 12 (STEP 4).

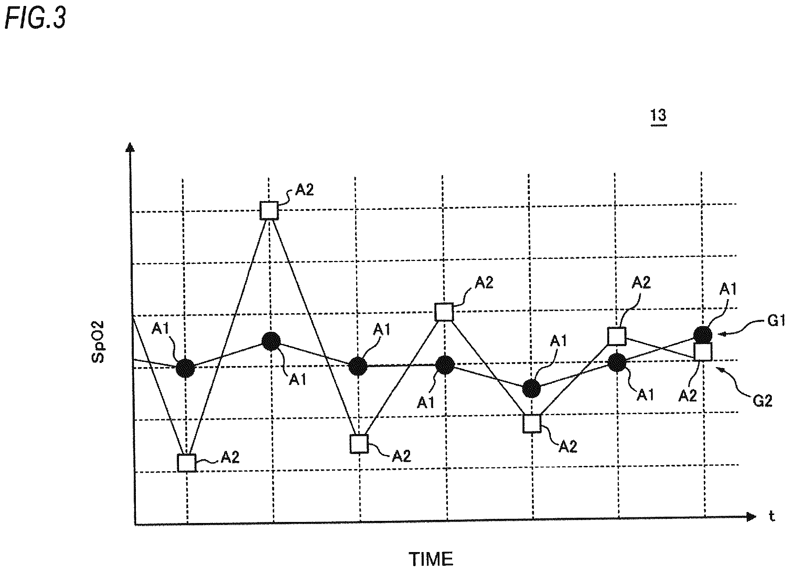

For example, each of the first and second time-averaged values A1, A2 is indicated by a point in the graph curve illustrated in FIG. 3. In FIG. 3, the first time-averaged value A1 is indicated by black circles, and the second time-averaged value A2 is indicated by white squares.

Then, the process returns to STEP 1, and the above-described process is periodically repeated. Accordingly, as illustrated in FIG. 3, a first graph curve G1 indicating a temporal change of the first time-averaged value A1 and a second graph curve G2 indicating a temporal change of the second time-averaged value A2 are displayed on the display 13. At least the latest first time-averaged value A1 may be displayed also as a numerical value.

According to the above-described configuration, the user can check, through the display 13, time changes of a plurality of time-averaged values which are calculated from the SpO2 that is acquired from the same subject, for respective different time periods. The second graph curve G2 indicating a temporal change of the second time-averaged value A2 which is calculated for the shorter second time period T2 reflects more strongly the variation of the acquired SpO2. Therefore, when the consistency between the first graph curve G1 and the second graph curve G2 is low, it is indicated that the SpO2 of the subject is unstable. In the example illustrated in FIG. 3, for example, the consistency between the first graph curve G1 and the second graph curve G2 becomes higher as time passes. Therefore, it is recognized that the SpO2 of the subject is gradually stabilized.

That is, the user can recognize not only the time-averaged value of the SpO2 of the subject, but also the stability of the SpO2, through the display 13. Therefore, it is possible to assist the user to accurately recognize the condition of the subject.

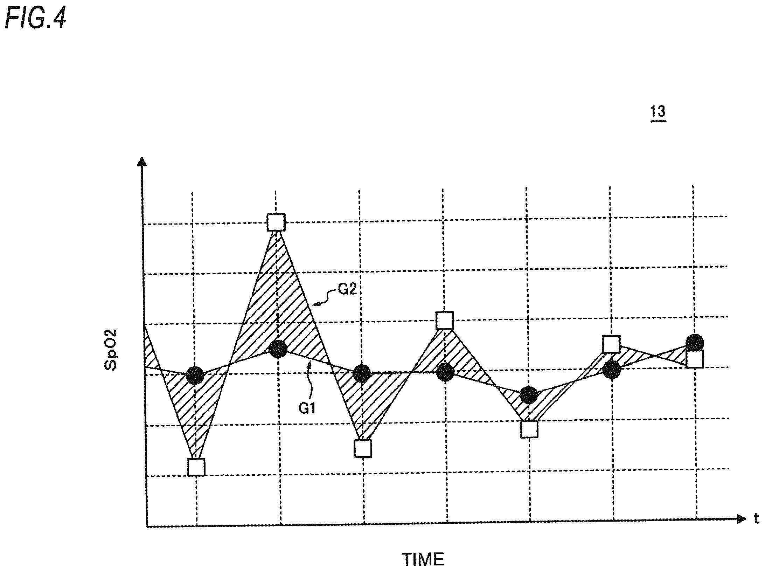

As illustrated in FIG. 4, the one or more regions which is located between the first graph curve G1 and the second graph curve G2 may be displayed in a color that is different from the background color of the display 13.

According to the above-described configuration, it is recognized that the larger the areas of the regions which are displayed in the different color, the higher the inconsistency between the first graph curve G1 and the second graph curve G2, and the lower the stability of the SpO2 value. Therefore, a change in condition of the subject can be recognized more intuitively.

The above-described function of the processor 12 may be realized by a general-purpose microprocessor which operates in cooperation with one or more memory, or by a dedicated integrated circuit such as a microcontroller, an FPGA (Field Programmable Gate Array) or an ASIC (Application Specific Integrated Circuit).

The above-described exemplary embodiment is a mere example for facilitating understanding of the presently disclosed subject matter. The configuration of the exemplary embodiment may be appropriately changed or improved without departing from the presently disclosed subject matter.

In the above-described exemplary embodiment, each of the first time-averaged value A1 and the second time-averaged value A2 is calculated as a simple average value. However, at least one of the first time-averaged value A1 and the second time-averaged value A2 may be acquired as the intermediate value or mode value of a plurality of SpO2 values which are acquired during a corresponding time period before the present time.

In the above-described embodiment, the first and second graph curves are displayed on the display. However, the first and second graph curves may be output (printed) on a sheet. Alternatively, data for displaying the first and second graph curves may be output to an external device such as a smartphone. According to this configuration, the first and second graph curves can be checked on a display of the external device.

The configuration which has been described above with reference to the exemplary embodiment can be applied to arbitrary physiological information which can be acquired from a subject and which involves time changes.

**Claims**

1. A physiological information measurement device comprising:

   one or more processors (12) configured to calculate a first time-averaged value (A1) of physiological information of a subject based for a first time period ending at a present time and calculate a second time-averaged value (A2) of the physiological information for a second time period that ends at the present time and is different from the first time period,
   wherein the first time-averaged value A1(t0) at the present time t0 is indicated by
   $$A1(t0) = \frac{1}{T1} \int_{t0-T1}^{t0} f(t)dt$$, where the time-varying physiological information is expressed as f(t) and T1 is the first time period,
   wherein the second time-averaged value A2(t0) at the present time t0 is indicated by
$$A2(t0) = \frac{1}{T2} \int_{t0-T2}^{t0} f(t)dt$$, where the time-varying physiological information is expressed as f(t) and T2 is the second time period; and
a display configured to display a first graph curve (G1) indicating a temporal change of the first time-averaged value (A1) and a second graph curve (G2) indicating a temporal change of the second time-averaged value (A2) simultaneously,
wherein the processor outputs data for displaying the first and second time-averaged values A1(t0), A2(t0) of the physiological information on the display.

2. The physiological information measurement device according to claim 1,
wherein a region which is located between the first graph curve and the second graph curve is displayed by a color that is different from a background color of the display.

3. A computer-implemented method for outputting data for displaying physiological information, the method comprising:

   acquiring physiological information of a subject;
   calculating a first time-averaged value (A1) of the physiological information for a first time period ending at a present time,
   wherein the first time-averaged value A1(t0) at the present time t0 is indicated by
   $$A1(t0) = \frac{1}{T1} \int_{t0-T1}^{t0} f(t)dt$$, where the time-varying physiological information is expressed as f(t) and T1 is the first time period;
   calculating a second time-averaged value (A2) of the physiological information for a second time period that ends at the present time and is different from the first time period,
   wherein the second time-averaged value A2(t0) at the present time t0 is indicated by
   $$A2(t0) = \frac{1}{T2} \int_{t0-T2}^{t0} f(t)dt$$, where the time-varying physiological information is expressed as f(t) and T2 is the second time period; and
   outputting data for displaying a first graph curve (G1) indicating a temporal change of the first time-averaged value (A1) and a second graph curve (G2) indicating a temporal change of the second time-averaged value (A2) simultaneously,
   wherein the output data are for displaying the first and second time-averaged values A1(t0), A2(t0) of the physiological information on a display.

**Patentansprüche**

1. Vorrichtung für Messung physiologischer Informationen, die umfasst:

einen oder mehrere Prozessor/en (12), der/die so konfiguriert ist/sind, dass er/sie einen ersten zeitlich gemittelten Wert (A1) physiologischer Informationen eines Patienten für einen ersten Zeitraum berechnet/berechnen, der zu einem aktuellen Zeitpunkt endet, und einen zweiten zeitlich gemittelten Wert (A2) der physiologischen Informationen für einen zweiten Zeitraum berechnet/berechnen, der zu dem aktuellen Zeitpunkt endet und sich von dem ersten Zeitraum unterscheidet,
wobei der erste zeitlich gemittelte Wert A1 (t0) zu dem aktuellen Zeitpunkt t0 durch

$$A1(t0) = \frac{1}{T1} \int_{t0-T1}^{t0} f(t)dt$$

dargestellt wird und dabei die zeitlich variierenden physiologischen Informationen als f(t) ausgedrückt werden und T1 der erste Zeitraum ist, wobei der zweite zeitlich gemittelte Wert A2(t0) zu dem aktuellen Zeitpunkt t0 durch

$$A2(t0) = \frac{1}{T2} \int_{t0-T2}^{t0} f(t)dt$$

dargestellt wird und dabei die zeitlich variierenden physiologischen Informationen als f(t) ausgedrückt werden und T2 der zweite Zeitraum ist; sowie
eine Anzeigeeinrichtung, die so ausgeführt ist, dass sie eine erste Kurve (G1) in einem Diagramm, die eine zeitliche Änderung des ersten zeitlich gemittelten Wertes (A1) darstellt, und gleichzeitig eine zweite Kurve (G2) in dem Diagramm anzeigt, die eine zeitliche Änderung des zweiten zeitlich gemittelten Wertes (A2) darstellt,
wobei der Prozessor Daten zum Anzeigen des ersten sowie des zweiten zeitlich gemittelten Wertes A1(t0), A2(t0) der physiologischen Informationen auf der Anzeigevorrichtung ausgibt.

2. Vorrichtung für Messung physiologischer Informationen nach Anspruch 1,
wobei ein Bereich, der sich zwischen der ersten Kurve in dem Diagramm und der zweiten Kurve in dem Diagramm befindet, mit einer Farbe angezeigt wird, die sich von einer Hintergrundfarbe der Anzeigeeinrichtung unterscheidet.

3. Computerimplementiertes Verfahren zum Anzeigen physiologischer Informationen, wobei das Verfahren umfasst:

Erfassen physiologischer Informationen eines Patienten;
Berechnen eines ersten zeitlich gemittelten Wertes (A1) der physiologischen Informationen für einen ersten Zeitraum, der zu einem aktuellen Zeitpunkt endet,
wobei der erste zeitlich gemittelte Wert A1(t0) zu dem aktuellen Zeitpunkt t0 durch

$$A1(t0) = \frac{1}{T1} \int_{t0-T1}^{t0} f(t)dt$$

dargestellt wird und dabei die zeitlich variierenden physiologischen Informationen als f(t) ausgedrückt werden und T1 der erste Zeitraum ist,
Berechnen eines zweiten zeitlich gemittelten Wertes (A2) der physiologischen Informationen für einen zweiten Zeitraum, der zu dem aktuellen Zeitpunkt endet und sich von dem ersten Zeitraum unterscheidet,
wobei der zweite zeitlich gemittelte Wert A2(t0) zu dem aktuellen Zeitpunkt t0 durch

$$A2(t0) = \frac{1}{T2} \int_{t0-T2}^{t0} f(t)dt$$

dargestellt wird und dabei die zeitlich variierenden physiologischen Informationen als f(t) ausgedrückt werden und T2 der zweite Zeitraum ist; sowie
Ausgeben von Daten zum Anzeigen einer ersten Kurve (G1) in einem Diagramm, die eine zeitliche Änderung des ersten zeitlich gemittelten Wertes (A1) darstellt, und gleichzeitig einer zweiten Kurve (G2) in dem Diagramm , die eine zeitliche Änderung des zweiten zeitlich gemittelten Wertes (A2) darstellt,
wobei die ausgegebenen Daten zum Anzeigen des ersten sowie des zweiten zeitlich gemittelten Wertes A1(t0), A2(t0) der physiologischen Informationen auf einer Anzeigeeinrichtung dienen.

**Revendications**

1. Dispositif de mesure d'informations physiologiques comprenant :

un ou plusieurs processeurs (12) configurés pour calculer une première valeur moyenne établie en fonction du temps (A1) d'informations physiologiques d'un sujet sur la base d'une première période de temps se terminant à un moment présent et pour calculer une seconde valeur moyenne établie en fonction du temps (A2) des informations physiologiques pour une seconde période de temps qui se termine au moment présent et qui est différente de la première période de temps,

dans lequel la première valeur moyenne établie en fonction du temps A1(t0) au moment présent t0 est indiquée par

$$A1(t0) = \frac{1}{T1} \int_{t0-T1}^{t0} f(t)\, dt$$

, où les informations physiologiques qui varient dans le temps sont exprimées par f(t) et T1 est la première période de temps,

dans lequel la seconde valeur moyenne établie en fonction du temps A2(t0) au moment présent t0 est indiquée par

$$A2(t0) = \frac{1}{T2} \int_{t0-T2}^{t0} f(t)\, dt$$

, où les informations physiologiques qui varient dans le temps sont exprimées par f(t) et T2 est la seconde période de temps; et

un affichage configuré pour afficher une première courbe de graphique (G1) indiquant un changement temporel de la première valeur moyenne établie en fonction du temps (A1) et une seconde courbe de graphique (G2) indiquant un changement temporel de la seconde valeur moyenne établie en fonction du temps (A2) simultanément,

dans lequel le processeur délivre des données pour l'affichage de la première et de la seconde valeur moyenne établie en fonction du temps A1(t0), A2(t0) des informations physiologiques sur l'affichage.

2. Dispositif de mesure d'informations physiologiques selon la revendication 1,
dans lequel une région qui est localisée entre la première courbe de graphique et la seconde courbe de graphique est affichée par une couleur qui est différente d'une couleur d'arrière-plan de l'affichage.

3. Procédé mis en œuvre par ordinateur pour délivrer des données pour l'affichage d'informations physiologiques, le procédé comprenant :

l'acquisition d'informations physiologiques d'un sujet ;
le calcul d'une première valeur moyenne établie en fonction du temps (A1) des informations physiologiques pour une première période de temps se terminant au moment présent,

dans lequel la première valeur moyenne établie en fonction du temps A1(t0) au moment présent t0 est indiquée par

$$A1(t0) = \frac{1}{T1} \int_{t0-T1}^{t0} f(t)\, dt$$

, où les informations physiologiques qui varient dans le temps sont exprimées par f(t) et T1 est la première période de temps;

le calcul d'une seconde valeur moyenne établie en fonction du temps (A2) des informations physiologiques pour une seconde période de temps qui se termine au moment présent et qui est différente de la première période de temps,

dans lequel la seconde valeur moyenne établie en fonction du temps A2(t0) au moment présent t0 est indiquée par

$$A2(t0) = \frac{1}{T2} \int_{t0-T2}^{t0} f(t)\, dt$$

, où les informations physiologiques qui varient dans le temps sont exprimées par f(t) et T2 est la seconde période de temps ; et

la délivrance de données pour l'affichage d'une première courbe de graphique (G1) indiquant un changement temporel de la première valeur moyenne établie en fonction du temps (A1) et simultanément d'une seconde courbe de graphique (G2) indiquant un changement temporel de la seconde valeur moyenne établie en fonction du temps (A2),

dans lequel les données délivrées servent à l'affichage de la première et de la seconde valeur moyenne établie en fonction du temps A1(t0), A2(t0) des informations physiologiques sur un affichage.

*FIG.1*

*FIG.2*

```
          ┌─────────────┐
          │    START    │
          └─────────────┘
                 │
                 ▼
      ┌──────────────────────┐
 ┌───→│   CALCULATE SpO2     │──── STEP1
 │    └──────────────────────┘
 │               │
 │               ▼
 │    ┌──────────────────────┐
 │    │  CALCULATE FIRST TIME-│──── STEP2
 │    │  AVERAGED VALUE OF SpO2│
 │    └──────────────────────┘
 │               │
 │               ▼
 │    ┌──────────────────────┐
 │    │ CALCULATE SECOND TIME-│──── STEP3
 │    │ AVERAGED VALUE OF SpO2│
 │    └──────────────────────┘
 │               │
 │               ▼
 │    ┌──────────────────────┐
 │    │OUTPUT DATA FOR DISPLAYING│── STEP4
 │    │ FIRST AND SECOND TIME-│
 │    │   AVERAGED VALUES     │
 │    └──────────────────────┘
 │               │
 └───────────────┘
```

FIG.3

EP 3 598 936 B1

*FIG.4*

EP 3 598 936 B1

13

**EP 3 598 936 B1**

<br>

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018139512 A
- JP 2015107229 A
- US 20110196213 A1
- US 20110004085 A1